(19) 

Europäisches
Patentamt
European
Patent Office
Office européen
des brevets

(11) **EP 2 340 888 A1**

(12) **EUROPEAN PATENT APPLICATION**
published in accordance with Art. 153(4) EPC

(43) Date of publication:
    **06.07.2011 Bulletin 2011/27**

(21) Application number: **09822101.3**

(22) Date of filing: **20.10.2009**

(51) Int Cl.:
    *B01J 27/198* (2006.01)    *B01J 37/08* (2006.01)
    *C07C 27/00* (2006.01)     *C07C 45/38* (2006.01)
    *C07C 47/22* (2006.01)     *C07C 51/235* (2006.01)
    *C07C 57/04* (2006.01)     *C07B 61/00* (2006.01)

(86) International application number:
    **PCT/JP2009/068302**

(87) International publication number:
    **WO 2010/047405 (29.04.2010 Gazette 2010/17)**

(84) Designated Contracting States:
    **AT BE BG CH CY CZ DE DK EE ES FI FR GB GR
    HR HU IE IS IT LI LT LU LV MC MK MT NL NO PL
    PT RO SE SI SK SM TR**
    Designated Extension States:
    **AL BA RS**

(30) Priority: **24.10.2008 JP 2008273889**

(71) Applicants:
    • **Nippon Kayaku Kabushiki Kaisha
      Tokyo 102-8172 (JP)**
    • **ARKEMA FRANCE
      92700 Colombes (FR)**

(72) Inventors:
    • **MAGATANI, Yasuhiro
      SanyoOnoda-shi
      Yamaguchi 757-8686 (JP)**
    • **OKUMURA, Kimito
      SanyoOnoda-shi
      Yamaguchi 757-8686 (JP)**
    • **DUBOIS, Jean-Luc
      Millery 69390 (FR)**

(74) Representative: **Benson, John Everett**
    **J. A. Kemp & Co.
    14 South Square
    Gray's Inn
    London WC1R 5JJ (GB)**

(54) **CATALYST FOR PRODUCTION OF ACROLEIN AND ACRYLIC ACID BY MEANS OF DEHYDRATION REACTION OF GLYCERIN, AND PROCESS FOR PRODUCING SAME**

(57)    A process for converting glycerin into valuable chemical raw material. A catalyst for use in the production of acrolein and acrylic acid by a dehydration reaction of glycerin, which enables the production of acrolein and acrylic acid in high yield. Glycerin dehydration catalyst which mainly comprises a phosphorus-vanadium complex oxide that contains phosphorus and vanadium as the essential constituent elements.

EP 2 340 888 A1

**Description**

**Technical Field**

This invention relates to a novel dehydration catalyst. In particular, this invention relates to a novel catalyst for producing acrolein and acrylic acid by catalytic dehydration of glycerin in gas phase or liquid phase, and its preparation method.

**Background Art**

Today's industrial production process for preparing unsaturated aldehyde and unsaturated carboxylic acids such as acrolein and acrylic acid is a gas phase catalytic oxidation process starting from propylene effected in the presence of a catalyst.

However, from the viewpoint of global warming and petroleum depletion, it is requested recently to develop another technique to produce fuels and organic products from bio resources that do not depend on fossil resources. Examples of such products are bio-ethanol and bio-diesel obtained by a conversion of fats and oils, the output of which may exceed 20 million ton/year. Since they are produced from vegetable oil, they can be an alternative fuel of fossil fuel and decrease discharge of carbon dioxide, so that their increase in demand is expected.

Glycerin is produced as a byproduct in a process for preparing the bio-diesel and the quantity of glycerin is such large as about 1/10 of the total output, resulting in that a large amount of glycerin is disposed in a form of industrial waste due to in balance between demand and supply and only a part of glycerin produced is utilized as materials for medicines, cosmetic and foodstuff additives. Therefore, new synthesis reactions are researched very actively to develop novel uses of glycerin and/or to expand its demand.

The conversion process of glycerin to chemical materials with keeping its skeleton of three carbons can be classified roughly into two reactions of dehydration reaction and oxidation reaction. In this invention, the dehydration reaction will be explained with reference to acrolein production but the oxidation reaction is not explained here.

Several processes for producing acrolein were already proposed. For example, in U.S. Patent No.2,558,520, acrolein is obtained with a yield of 72% by using a catalyst of phosphoric acid supported on a carrier such as diatomaceous earth. The catalyst is dispersed in an organic solvent having a high boiling point in to which glycerin is added drop-wise to effect liquid-phase dehydration reaction. This process, however, may not be used in industrial scale, because a large amount of carbides is produced and a separation stage for a liquid mixture comprising the carbide, catalyst and products is necessary.

JP-A1-2006-290815 discloses a process for producing acrolein in which glycerin is dispersed in a solvent and is subjected to a liquid-phase dehydration reaction in the presence of an acid solid catalyst possessing $H_0$ of -5.6 to +3.3 such as $KHSO_4$ and $K_2SO_4$, In this process also, more than 10% of carbides is produced, so that the process can hardly say as an industrial sufficient process and improvement is necessary.

When acrolein is produced by a liquid-phase or gas-phase contact reaction of glycerin, solid acid catalysts possessing $H_0$ of less than +2 are effective. An example of such solid acid catalysts is phosphoric acid catalyst supported on $\alpha$-$Al_2O_3$ as is shown in JP-A1-6-211724. In this patent, acrolein is obtained at a yield of 75% in gas-phases at 300°C but the life of catalyst is short. Still more, amounts of by-products such as hydroxy acetone increase in time and the selectivity also is not so high.

WO2007/058221 discloses a process for producing acrolein by a dehydration reaction of glycerin in gas-phase in the presence of heteropolyacid used as a solid acid catalyst. The heteropolyacid is those of Group 6 element such as tungstosilicic acid, tungstophosphoric acid and phosphomolybdic acid. These heteropolyacids are supported on dual-pores silica carrier and produce acrolein at a yield of 86%. This dehydration reaction of the glycerin, however, is effected with free of oxidation gas but using nitrogen stream as carrier gas, so that deposition of carbon increase seriously and hence there is a problem of deterioration in time of stability, activity and selectivity of the catalysis.

In WO20061087fl83 and WO2006/087084, oxygen is introduced to prevent degradation of the catalyst in the gas-phase reaction of glycerin. In this patent, the catalyst possessing the acid strength of $H_0$ of -9 to -18 is used. A variety of solid acid catalysts such as phosphoric acid/zirconia, Nafion/silica, sulfuric acid/zirconia, tungsten/zirconia are used in Examples and the highest yield of acrolein of 74% was obtained when tungsten/zirconia catalyst was used.

Complex oxide catalysts of phosphorus-vanadium system which is used in this invention are widely known as an effective catalyst to prepare maleic anhydride by gas-phase catalytic oxidation reaction of hydrocarbon having a carbon number of 4 such as n- butane, 1-butene, 2-butene and 1,3-butadiene. In particular, for n-butane that is less reactive, a crystalline compound represented by the general formula: $(VO)_2P_2O_7$ (divanadyl pyrophosphorate) which is a structure having higher activity is used as a catalyst component (E. Bordes, P. Courtine, J. Catal., 57236-252, 1979). An active component: $(VO)_2P_2O_7$ of the catalyst can be obtained, for example, by reacting $V_2O_5$ (vanadium pentoxide) with $H_3PO_4$ (phosphoric acid) in an organic solvent or alcohol such as 2-methyl-1-propanol to precipitate $VOHPO_4$

·1/2H$_2$O which is a precursor the divanadyl pyrophosphorate, followed by firing and dehydration under suitable conditions. A variety of studies were made to improve the catalytic activity and selectivity of this catalyst. For example, a variety of promoters are added to the phosphorus-vanadium complex oxide. Such promoters are summarized in Burnett et al. Catal. Today, 1537 (1987).

JP-A1-54-30114 discloses a process for depositing metal elements such as magnesium and calcium as an accelerator on a surface of phosphorus-vanadium system compound oxide.

JP-A1-57-24643 discloses a ring-shaped catalyst consisting of vanadium-phosphorus system compound and metal elements such as copper; silver and zinc are mentioned as addition metal to improve the activity.

JP-A1-58-114735 discloses a process for activating the vanadium-phosphorus system catalyst with oxygen and reducing gas and alkali metal, alkaline earth metal, lanthanide are mentioned as promoter component.

JP-A1-59-12759 discloses a technique to add water to an organic slurry including catalyst precursor to separates it into two phases and then to recover the precursor and alkali metal, alkaline earth metal, lanthanide are mentioned as promoter component.

JP-A 1-59-145046 discloses a process for preparing a catalyst by mixing a crystalline vanadium-phosphorus compound having a specific X-ray diffraction spectrum, an aqueous solution of vanadyl phosphorate, a compound of at least one element selected from magnesium, calcium, strontium and barium and silica, followed by drying.

JP-A1-4-271841 discloses a process for preparing a precursor by reacting vanadium penta oxide with phosphoric acid in the presence of Mg acetyl acetonato or Zr acetyl acetonato.

JP-A1-7-171398 discloses a process for preparing a precursor by reacting a phosphorus compound of penta valence with a vanadium compound of penta valence in a mixture of aliphatic alcohol having a carbon number of 3 to 6 and benzyl alcohol added with iron oxalate dihydrate as promoter.

JP-A1-9-52049 discloses a process for preparing a catalyst by reacting vanadium pentoxide with phosphoric acid, added with alkyl silanol that is an organic silicon compound.

JP-A1-10-87308 discloses a process for preparing metal ion exchanged VOM$_{0.5}$PO$_4$ which is a phosphorus-vanadium system compound in which H$^+$ positioned at an intercalation of catalyst precursor VOHPO$_4$ · 1/2H$_2$O is exchanged by divalent metal cation such as Co.

As shown as above, the phosphorus-vanadium system compound oxides were reported in many literatures as catalysts to produce dicarboxylic anhydride from hydrocarbon having a carbon number of 4 at high yield and are actually industrialized. However, there is no report to use as a catalyst for producing acrolein and acrylic acid in dehydration reaction of glycerin.

## Disclosure of Intention

### Technical Problems

Therefore, an object of this invention is to provide a novel dehydration catalyst, in particular to a catalyst that can produce acrolein and acrylic acid at high yield from glycerin which is a material that does not derive from petroleum.

Another object of this invention is to provide a catalyst for glycerin dehydration reaction in which glycerin is dehydrated to produce acrolein and acrylic acid at high yield.

Still another object of this invention is to provide a method for preparing the catalyst.

### Technical Solution

Inventors made deep study to solve the above problem and found that acrolein and acrylic acid can be obtained by the dehydration reaction of glycerin at high yield by using phosphorus-vanadium complex oxide containing phosphorus and vanadium as indispensable constituent elements, and completed this invention.

The present invention is characterized by following features of (1) to (8) separately or in combination:

(1) A catalyst for glycerin dehydration used in preparation of acrolein and acrylic acid by catalytic dehydration reaction of glycerin, comprising, as a main constituent, phosphorus-vanadium complex oxides or their precursors, containing as an indispensable element, phosphorus and vanadium.

(2) The catalyst of (1), wherein the phosphorus-vanadium complex oxides have the composition represented by the general formula (I):

VPaMbOc nH$_2$O          (I)

in which, when V is considered as 1, a and b satisfy respective ranges of $0.5 \leqq a \leqq 1.5$ and $0 < b \leqq 1$, c has a number which depends on the oxidation state of each element, M is an element selected from hydrogen atom or elements belonging to Group 1 to Group 16 of the Periodic Table, n is a arbitrary positive number.

(3) The catalyst of (1) or (2), wherein a precursor of the phosphorus-vanadium complex oxide is a substance whose at least a part becomes $(VO)_2P_2O_7$ by firing.

(4) A supported catalyst in which the phosphorus-vanadium complex oxide of any one of (1) to (3) is supported on a carrier.

(5) A process for preparing the phosphorus-vanadium complex oxide catalyst according to any one of (1) to (3), characterized by the steps of reacting vanadium compound and phosphorus compound in an aqueous solvent or in an organic solvent to prepare phosphorus-vanadium complex oxide, and subjecting the oxide to drying and firing.

(6) The process of (5), the firing is effected in an atmosphere that contains any one of gases of air or inert gas or a mixed gas of air and inert gas or a mixed gas of reducing gas and air or steam.

(7) The process of (5) or (6), the firing is carried out at 150 to 700°C for 0.5 to 500 hours.

(8) Use of the catalyst of any one of claims (1) to (3) and/or the supported catalyst of (4) in preparation of acrolein and acrylic acid by the catalytic dehydration reaction of glycerin.

## Advantageous Effect

The catalyst for producing acrolein and acrylic acid for catalytic dehydration reaction of glycerin according to this invention has a high activity and high selectivity. By-products such as propion aldehyde and propionic acid are formed in reduced or lower amounts than the conventional solid acid catalysts used usually in this reaction. This makes the present invention advantageous in industrial uses, because propion aldehyde and propionic acid have their boiling points of respectively 49°C and 141°C which are very close to boiling points of 53°C and 141°C respectively of the objective compounds of acrolein and acrylic acid and hence which make purification operation much difficult.

## Best Made for Carrying out the Invention

The dehydration catalyst of this invention is a catalyst used to produce acrolein and acrylic acid by dehydration of glycerin and consisting mainly of phosphorus-vanadium complex oxide. The phosphorus-vanadium complex oxide may be prepared from a precursor of $VOHPO_4 \cdot 1/2H_2O$ crystal which is obtained generally by the above-mentioned reaction between $V_2O_5$ and $H_3PO_4$, as a deposit.

The precursor can be prepared by any method reported and it is preferable to use one of following three methods (a) to (c). Here, we explain phosphorus-vanadium complex oxides consisting of phosphorus and vanadium. The phosphorus-vanadium complex oxide however may contain other element(s) belonging to the groups 1 to 16, provided that the complex oxide contains phosphorus and vanadium as metal elements.

(a) Phosphoric acid is added before or after vanadium pentoxide is reduced in a heated reducible organic solvent such as 2-methyl-1-propanol, and the reaction mixture is refluxed under heat to obtain vanadyl hydrogen phosphate semi-hydride $VOHPO_4 \cdot 1/2H_2O$ (see JP-B1-57-8761).

(b) Phosphoric acid is added before or after vanadium pentoxide is reduced in an inorganic reducing agent such as hydrochloric acid or hydrazine and the reaction mixture is subjected to refluxing under heat or to treatment under hydrothermal condition (see JP-A1-57-32110).

(c) $VOPO_4 \cdot 0.5H_2O$ is heat-refluxed in a reducing organic solvent such as 2-methyl-1-propanol and 2-propanol ("Catalysis Today", 33 (1997), 161-171, Chem. Mater. 2002, 14, 3882-3888)

Now, the methods (a), (b) and (c) which are desirable in this invention will be explained in details.

A vanadium source used to prepare the precursor can be any vanadium compound that is usually used for the preparation of phosphorus-vanadium complex oxide and may be penta, tetra or tri valence vanadium compound such as vanadium pentoxide, metavanadinic acid salts and oxyhalogenated vanadium. Among them, vanadium pentoxide is preferably used.

As phosphorus source, like vanadium source, it is possible to use phosphorus compounds used usually in preparation of phosphorus-vanadium complex oxides and may be orthophosphoric acid, pyrophosphoric acid, phosphorous acid, polyphosphoric acid and phosphorus pentoxide. Among them, orthophosphoric acid is preferably used. Commercially available 85 wt% phosphoric acid can be used but it is desirable to use substantially anhydrous phosphoric acid in order to produce a catalyst that can produce acrolein and acrylic acid at high yield. The substantially anhydrous is understood that the content of phosphoric acid in term of orthophosphoric acid $H_3PO_4$ is more than 95 wt%, preferably more than 98 wt%.

A mixing ratio of phosphorus compound to vanadium compound (P/V mole ratio) is usual 0.5 to 1.5 and preferably 1.0 to 1.3.

The catalyst for producing acrolein and acrylic acid by catalytic dehydration reacting of glycerin is phosphorus-vanadium complex oxide containing phosphorus and vanadium as indispensable constituent elements and represented by the general formula (I):

$$VPaMbOc \cdot nH_2O \qquad (I)$$

in which, when V is considered as 1, a and b satisfy respective ranges of $0.5 \leqq a \leqq 1.5$ and $0 < b \leqq 1$, c has a number which depends on the oxidation state of each element, M is an element selected from hydrogen atom or elements belonging to Group 1 to Group 16 of the Periodic Table, n is a arbitrary positive number.

The elements belonging to Group 1 to Group 16 of the Periodic Table may be sodium, potassium, rubidium, cesium, magnesium, calcium, strontium, barium, scandium, yttrium, lanthanide, titanium, zirconium, hafnium, chromium, manganese, rhenium, iron, ruthenium, osmium, cobalt, nickel, palladium, platinum, copper, silver, gold, zinc, gallium, thallium, germanium, tin, leads, bismuth and tellurium. Compounds of the elements belonging to Group 1 to Group 16 of the Periodic Table used may be metal salts and onium salts. The onium salts may be amine salt, ammonium salt, phosphonium salt and sulfonium salt. Materials for the metal salts and onium salts can be nitrate, carbonate, sulfates, acetates, oxides, halide and hydroxides of the metal or onium but they are not limited to them. A proportion is 0.001 % by weight to 60 % by weight, preferably 0.01 % by weight to 30 % by weight in term of the % by weight of metal in metal salts and of onium ion onium salts with respect to the phosphorus-vanadium complex oxide.

Solvent used is not limited especially and can be organic solvents and water-soluble solvent. Preferably, the solvent is those that can reduce vanadium when heated. Organic solvent is preferably those having alcoholic hydroxyl group such as aliphatic alcohol having a carbon number of 3 to 5 like 2-propanol and 2-methyl-1-propanol and aromatic alcohol such as benzyl alcohol. The water-soluble solvent is aqueous solution of a compound that can reduce vanadium when heated, such as aqueous solutions of hydrochloric acid, nitric acid, hydrazine and oxalic acid. The solvent can be a mixture. For example, it is possible to use a mixture of 2-propanol or 2-methyl-1-propanol added with benzyl alcohol having relatively higher reducing power or a mixture of an aqueous solution of oxalic acid and an aqueous solution of hydrazine. It is also possible to use a mixture of non-aqueous solvent and water-soluble solvent. An amount of solvent is not limited specially, provided that it is a quantity that can be used as reaction medium. For example, when 2-methyl-1-propanol is used, a mole ratio of 2-methyl-1-propanol to vanadium compound is usual 10:0.1 to 1:1, preferably 5:0.1 to 1:0.1.

In the reaction, vanadium compound is added to the solvent and the mixture is heated to reduce vanadium. Addition of the phosphorus compound and the compound of element belonging to Group 1 to Group 16 can be done after the reduction of vanadium is advanced to some extent, or from the start of reaction so that the phosphorus compound is reacted while vanadium is reduced. The reduction of vanadium can be effected for example by heating it in an aliphatic alcohol having a carbon number of 3 to 5 and aromatic alcohol such as benzyl alcohol. Temperature of the reaction (reduction step of vanadium and a reaction step with phosphorus compound) depends on a type of solvent used and is usually 80 to 200°C. The reaction time after the addition of phosphorus compound is usually 1 to 20 hours.

After the reaction completes, a slurry containing, as active structure, mainly a crystal structure of $VOHPO_4$ $\cdot 1/2H_2O$, or a slurry containing further other metals, or a slurry deposited on a carrier is obtained. The slurry is then subjected to evaporation to dryness, spray drying, centrifugal separation, filtration or the like to isolate a precursor. The precursor isolated is washed with a volatile organic solvent such as acetone and can be dried by suitable means.

The precursor obtained can be used as it is or is subjected to activation so as to exhibit an activity in an objective reaction, so that it can be used as catalyst in this invention. The precursor can be fired to effect further activation treatment, resulting in that a proportion of the phosphorus-vanadium composite metal oxide consisting mainly of $(VO)_2P_2O_7$ is advantageously increased.

The firing or calcination is carried out in an atmosphere of inert gas such as nitrogen and argon, or in air, or in a mixture of air containing reducing gas such as hydrocarbon, or in a mixed gas thereof containing further steam. A firing furnace is not limited specially and can be Muffle furnace, rotary kiln and fluidized bed firing furnace. The firing can be carried out in a reaction tube used as a reactor. The firing temperature is usually 150 to 700°C, preferably 300 to 600°C, more preferably 350 to 550°C. The firing time duration is preferably 0.5 to 500 hours.

The activation of precursor or activation of fired precursor is effected by passing air containing 1 to 70 vol%, preferably 5 to 40 vol% of hydrocarbon having a carbon number of more than 3 such as glycerin, butane, 1-butene, 2-butene and 1,3-butadiene or passing a mixed gas there of containing further steam under heating. The activation temperature is 150 to 700°C, preferably 300 to 600°C, more preferably 350 to 550°C. Elevation to the activation temperature or elevation speed to and cooling speed from the reaction temperature is not limited specially. The activation can be effected at ambient pressure or is between 0.05 and 10 kg/cm$^2$G. The space velocity (GHSV) is 100 to 10000 hr$^{-1}$, preferably 300 to 5000 hr$^{-1}$ in term of the volume of phosphorus-vanadium complex oxide catalyst. The activation time is preferably between 5 and 500 hours.

Glycerin dehydration catalyst according to this invention is used to dehydrate glycerin to obtain acrolein and acrylic acid and can be supported on a carrier (supported catalyst of this invention). The carrier may be silica, diatomaceous earth, alumina, silica alumina, silica magnesia, zirconia, titania, magnesia, zeolite, silicon carbide and carbide. The catalyst can be supported on one of these carriers or on a complex of more than two carriers or on a mixture of these carriers. By supporting on a carrier, By preserving carrier, the active material can be used effectively and sintering of the active components can be avoided advantageously so that the life of catalyst is improved. An amount of the phosphorus-vanadium complex oxide catalyst on the carrier is 5 to 200 % by weight, preferably 10 to 150 % by weight.

Shape of the catalyst is not limited specially and can be granule and powder. In the gas phase reaction, the catalyst is shaped into spheres, pellets, cylindrical body, hollow cylinder bodies and bars with optional adding a molding aid. Or, the catalyst is mixed with carrier and other auxiliary components and is shaped into the above configurations with optional adding a molding aid. The molded catalyst has preferably, for example in case of a sphere, a particle size of 1 to 10mm for fixed bed catalyst, and a particle size of less than 1 mm for fluidized bed catalyst-

The glycerin dehydration reaction according to this invention can be effected both of in gas phase and in liquid phase but gas phase is desirable. The gas phase reaction can be carried out in a variety of reactors such as fixed bed, fluidized bed, circulating fluidized bed and movable bed. Among them, the fixed bed is preferable. Regeneration of the catalyst can be done inside or outside the reactor. When the regeneration is effected outside the reactor, the catalyst is taken out of the reactor and is burn in air or in an oxygen-containing gas. The liquid phase reaction can be carried out in a reactor that is used generally in liquid phase reaction for solid catalyst. The operation is effected at relatively lower temperature so that acrolein produced can be distilled out continuously, since difference in boiling points between unsaturated aldehyde and unsaturated carboxylic acid and glycerin (290°C) is very big.

A reaction temperature for the dehydration reaction of glycerin in gas phase according to this invention is preferably at 200 to 450°C. In fact, since glycerin has a high boiling point, the life of catalyst will be shortened due to polymerization and carbonization of glycerin and reaction products if the reaction temperature becomes lower than 200°C. On the other hand, if the reaction temperature exceeds 450°C, the selectivity of acrolein and acrylic acid will be lowered due to parallel reactions and successive reactions. A preferred reaction temperature is 250°C to 350°C. The reaction pressure is not specially limited but is preferably lower than 2 atm, preferably lower than 1 atm. In fact, gasified glycerin will be re-liquefied under a higher pressure. In addition, depositions of carbon increase and hence the life of catalyst is shortened at higher pressure.

A feed rate of the material gas is 100 to 1000h$^{-1}$ in term of the space velocity GHSV to the catalyst. If the GHSV is not higher than 100h$^{-1}$, the selectivity will be lowered due to successive reactions and if the GHSV exceeds 1000h$^{-1}$, the conversion of glycerin will be lowered.

The liquid phase reaction is carried out desirably at 150°C to 350°C. The conversion will be lowered at lower temperatures but the selectivity is improved. The pressure is not limited specially. The reaction may be carried out under a pressurized condition under 3 atm to 70 atm.

The raw material of glycerin is available easily in a form of an aqueous solution of glycerol. A concentration of the aqueous solution of glycerol is preferably in a range of 5 to 90 % by weight, more preferably 10 to 50 % by weight. Higher concentration of glycerol is not desirable because, if the concentration of glycerol becomes higher, glycerin ether is formed and/or unsaturated aldehyde and unsaturated carboxylic acid produced react with glycerin. Still more, enormous energy is necessary to gasify glycerin.

The present invention will be explained more in detail by Examples. However, the invention should not be limited to the Examples. In the Examples, % means % by weight.

## Examples

### Examples 1

Catalyst used was phosphorus-vanadium complex oxide: VOHPO$_4$ · 1/2H$_2$O prepared by the exfoliation-reduction method which is described in Chem. Mater. 2002, 14, 3882-3888, Applied catalysis A: General 297(2006) 73-80 (Hokkaido Univ. Toshio OKUHARA et al). In practice, a mixture of vanadium pentoxide and 85% of phosphoric acid was stirred under reflux at 388K for 16 hours. The resulting sediment was filtered, washed with acetone and dried at ambient temperature. By the analysis of XRD and IR, it was conformed that the product has a structure of VOPO$_4$ · 2H$_2$O. This compound of VOPO$_4$ · 2H$_2$O was heated under stirring in 2-butanol to effect intercalation of 2-butanol into layers of VOPO$_4$ · 2H$_2$O, so that the VOPO$_4$ · 2H$_2$O layer is exfoliated. In to the resulting slurry, 2-propanol was added and stirred under reflux for 24 hours. The product was filtered, washed with acetone and dried at ambient temperature to obtain a blue white powder. This powder was fired at 550°C for 4 hours in nitrogen atmosphere.

The resulting catalyst was evaluated in a fixed bed type reactor at ambient pressure. The catalyst powder was compressed, crushed and then passed through a sieve to obtain particles of 50 to 80 mesh. A SUS reaction tube of 10 mm diameter was filled with 2 cc of the catalyst particles. An aqueous solution containing 40 % by weight of glycerol was passed to an evaporator heated at 300°C by a pump. The resulting gasified glycerin gas was directly passed through the catalyst together with oxygen and steam. The reactor containing the catalyst was heated at 280°C. The feed stream has a composition of glycerin: oxygen: water = 10 mol %: 10 mol %: 79 mol % and GHSV was 330h$^{-1}$.

The product was collected in a condenser and the resulting condensate was analyzed quantitatively by GC-MS (GC-17A + GC/MS-QP5050A, a product of Shimadzu, TC-WAX column, a product of GL Science). Each product was corrected in factor by this gas chromatograph. From amounts of glycerin fed, of residual glycerin and absolute content of each product, the conversion of material ratio (glycerin conversion), the selectivity of product (the selectivity of acrolein, acrylic acid, propionic acid and propionaldehyde), and the yield of product

(yields of acrolein, acrylic acid, propionic acid and propionaldehyde) were calculated by following equations:

$$\text{Conversion (\%) =} \quad \text{mole number of material reacted / mole number of material fed)} \times 100.$$

$$\text{Selectivity (\%) =} \quad \text{(mole number of objective product / mole number of material reacted)} \times 100.$$

$$\text{Yield (\% ) =} \quad \text{(mole number of objective product / mole number of material fed)} \times 100.$$

Results are summarized in Table 1.

Table 1

| | Catalytic | Reaction temp. (°C) | Glycerin conversion ratio (%) | Acrolein yield (%) | Acrylic acid yield (%) | Propion aldehyde Yield (%) | Propionic acid yield (%) |
|---|---|---|---|---|---|---|---|
| Examples 1 | VPO (after firing in nitrogen) | 280 | 100 | 79.8 | 0.5 | 0.0 | 0.0 |

Example 2

A precursor of $VOHPO_4 \cdot 1/2H_2O$ was prepared by a method described in JP-B1-57-8761. Namely, 100.0 g of vanadium pentoxide ($V_2O_5$) was suspended in 1000ml of 2-methyl-1-propanol and refluxed at 105°C under agitation for 3 hours to reduce $V_2O_5$. In 132.0 g of 98% orthophosphoric acid powder, 250 ml of 2-methyl-1-propanol was added and dissolved under agitation at 100 °C.

The resulting orthophosphoric acid solution (132.0 g of 98% orthophosphoric acid powder / 250ml of 2-methyl-1-propanol) was added at 100° C gradually into a yellow solution of vanadium prepared by the heat-reflux in 2-methyl-1-propanol and heat-reflux was continued at 105°C. After 3 hours, the reflux was stopped and cooled down to ambient temperature. The resulting catalyst precursor was filtered, washed with acetone and dried in a drier at 140°C during one night to obtain a blue white phosphorus-vanadium complex oxide powder.

The resulting catalyst was evaluated in a fixed bed type reactor at ambient pressure. The catalyst powder was compressed, crushed and then passed through a sieve to obtain particles of 9 to 12 mesh. A SUS reaction tube of 10 mm diameter was filled with 10 cc of the catalyst particles. An aqueous solution containing 20 % by weight of glycerol was passed to an evaporator heated at 300°C at a rate of 21 g/hr by a pump. The resulting gasified glycerin gas was directly passed through the catalyst together with air. The reactor containing the catalyst was heated at 300 to 340°C. The feed stream has a composition of glycerin: oxygen: nitrogen: water = 4.2 mol %: 2.2 mol %: 8.1 mol %: 85.5 mol % and GHSV was 2445 h$^{-1}$.

The product was collected in a condenser and the resulting condensate was analyzed quantitatively by gas chromatograph (GC-7890, DB-WAX column, product of Agilent). Each product was corrected in factor by this gas chromatograph. From amounts of glycerin fed, of residual glycerin and absolute content of each product, the conversion of material ratio (glycerin conversion), the selectivity of product (the selectivity of acrolein, acrylic acid, propionic acid and propionaldehyde), and the yield of product (yields of acrolein, acrylic acid, propionic acid and propionaldehyde) were calculated by following equations:

$$\text{Conversion (\%)} = \text{mole number of material reacted / mole number of material fed)} \times 100.$$

$$\text{Selectivity (\%)} = \text{(mole number of objective product / mole number of material reacted)} \times 100.$$

$$\text{Yield (\%)} = \text{(mole number of objective product / mole number of material fed)} \times 100.$$

Results are summarized in Table 1.

Example 3

Blue white dry powder obtained in Example 2 was fired in Muffle furnace in air atmosphere at 500°C for 3 hours to obtain a light green phosphorus-vanadium complex oxide powder.
Reactivity of this powder was evaluated by the same method as Example 2. Results are shown in Table 2.

Table 2

| | Catalytic | Reaction temp. (°C) | Glycerin conversion ratio (%) | yield (%) | Acrolein Acrylic acid yield (%) | Propion aldehyde yield (%) | Propionic acid yield (%) |
|---|---|---|---|---|---|---|---|
| Example 2 | VPO (precursor) | 300 | 100 | 49.5 | 0.2 | 0.4 | 0.2 |
| | | 340 | 100 | 42.5 | 0.3 | 0.8 | 0.0 |
| Example 3 | VPO (after firing in air) | 300 | 100 | 54.2 | 0.2 | 0.5 | 0.1 |
| | | 340 | 100 | 38.1 | 0.0 | 1.0 | 0.0 |

Comparative Example

For a comparison with the phosphorus-vanadium complex oxide, phosphoric acid alumina (1 wt%$PO_4$ /99wt% $Al_2O_3$) as a solid acid was evaluated.
The phosphoric acid alumina was prepared by a method described in JP-A1-2005-213225. Namely, 4 g of phosphoric acid was added to 2 g of Snowtex O (product of Nissan Chemical Industries) and mixed. Into the mixture, 194 g of $\alpha$-alumina and 200 ml of water were added and stirred at 80°C. The resulting white slurry was evaporated in a rotary evaporator at 80°C, and finally dried at 100°C for 6 hours.
Reactivity of the powder was evaluated by the same method as Example 2. Result is shown in following Table 3.

Table 3

| | Catalytic | Reaction temp. (°C) | Glycerin conversion ratio (%) | Acrolein yield (%) yield (%) | Acrylic acid yield (%) yield (%) | Propion aldehyde yield (%) | Propionic acid yield (%) |
|---|---|---|---|---|---|---|---|
| Comparative Example | $H_3PO_4$/ $Al_2O_3$ | 350 | 92.7 | 37.8 | 0.2 | 0.6 | 0.1 |

From the results of Examples and Comparison Example, following points are clear:

(1) Total yield of acrolein and of acrylic acid when acrolein and acrylic acid are prepared by dehydration reaction of glycerin can be elevated to about 80.3% at maximum by using the phosphorus-vanadium complex oxide of this invention.

(2) Yield of propionaldehyde or propionic acids, which make a separation operation very difficult, can be lowered and can be reduced to 0 % by selecting alcohol or by adjusting reaction conditions.

**Claims**

1. A catalyst for glycerin dehydration used in preparation of acrolein and acrylic acid by catalytic dehydration reaction of glycerin, comprising, as a main constituent, phosphorus-vanadium complex oxides or their precursors, containing, as an indispensable element, phosphorus and vanadium.

2. The catalyst according to claim 1, wherein said phosphorus-vanadium complex oxides have the composition represented by the general formula (I):

   $$VP_aM_bO_c \, nH_2O \qquad (I)$$

   in which, when V is considered as 1, a and b satisfy respective ranges of $0.5 \leqq a \leqq 1.5$ and $0 < b \leqq 1$, c has a number which depends on the oxidation state of each element, M is an element selected from hydrogen atom or elements belonging to Group 1 to Group 16 of the Periodic Table, n is a arbitrary positive number.

3. The catalyst of claim 1 or 2, wherein a precursor of said phosphorus-vanadium complex oxide is a substance whose at least a part becomes $(VO)_2P_2O_7$ by firing.

4. A supported catalyst in which said phosphorus-vanadium complex oxide of any one of claims 1 to 3 is supported on a carrier.

5. A process for preparing the phosphorus-vanadium complex oxide catalyst according to any one of claims 1 to 3, **characterized by** the steps of reacting vanadium compound and phosphorus compound in an aqueous solvent or in an organic solvent to prepare phosphorus-vanadium complex oxide, and subjecting the oxide to drying and firing.

6. The process of claim 5, said firing is effected in an atmosphere that contains any one of gases of air or inert gas or a mixed gas of air and inert gas or a mixed gas of reducing gas and air or steam.

7. The process of claim 5 or 6, said firing is carried out at 150 to 700°C for 0.5 to 500 hours.

8. Use of the catalyst of any one of claims 1 to 3 and/or of the supported catalyst of claim 4, in preparation of acrolein and acrylic acid by the catalytic dehydration reaction of glycerin.

**INTERNATIONAL SEARCH REPORT**

| | International application No. |
|---|---|
| | PCT/JP2009/068302 |

**A. CLASSIFICATION OF SUBJECT MATTER**
*B01J27/198*(2006.01)i, *B01J37/08*(2006.01)i, *C07C27/00*(2006.01)i, *C07C45/38* (2006.01)i, *C07C47/22*(2006.01)i, *C07C51/235*(2006.01)i, *C07C57/04* (2006.01)i, *C07B61/00*(2006.01)n
According to International Patent Classification (IPC) or to both national classification and IPC

**B. FIELDS SEARCHED**

Minimum documentation searched (classification system followed by classification symbols)
B01J27/198, B01J37/08, C07C27/00, C07C45/38, C07C47/22, C07C51/235, C07C57/04, C07B61/00

Documentation searched other than minimum documentation to the extent that such documents are included in the fields searched
| Jitsuyo Shinan Koho | 1922-1996 | Jitsuyo Shinan Toroku Koho | 1996-2010 |
|---|---|---|---|
| Kokai Jitsuyo Shinan Koho | 1971-2010 | Toroku Jitsuyo Shinan Koho | 1994-2010 |

Electronic data base consulted during the international search (name of data base and, where practicable, search terms used)
JSTPlus/JMEDPlus/JST7580(JDreamII), CAplus(STN), REGISTRY(STN)

**C. DOCUMENTS CONSIDERED TO BE RELEVANT**

| Category* | Citation of document, with indication, where appropriate, of the relevant passages | Relevant to claim No. |
|---|---|---|
| X<br>A | WO 2008/066079 A1 (Nippon Shokubai Co., Ltd.),<br>05 June 2008 (05.06.2008),<br>paragraphs [0030], [0037]; claims<br>& EP 2103591 A1 | 1,2,4-8<br>3 |
| X<br>A | JP 2005-213225 A (Nippon Shokubai Co., Ltd.),<br>11 August 2005 (11.08.2005),<br>claims; paragraph [0016]; example 5<br>& US 2007/0129570 A1 & EP 1710227 A1<br>& WO 2005/073160 A1 & CN 1910128 A | 1,2,4,8<br>3,5-7 |
| A | WO 2006/114506 A1 (ARKEMA FRANCE),<br>02 November 2006 (02.11.2006),<br>entire text<br>& JP 2008-538781 A & US 2008/0183013 A1<br>& EP 1874720 A & KR 10-2007-0116655 A<br>& CN 101248033 A | 1-8 |

| ☒ Further documents are listed in the continuation of Box C. | ☐ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 13 January, 2010 (13.01.10) | 26 January, 2010 (26.01.10) |

| Name and mailing address of the ISA/ | Authorized officer |
|---|---|
| Japanese Patent Office | |
| Facsimile No. | Telephone No. |

Form PCT/ISA/210 (second sheet) (April 2007)

**EP 2 340 888 A1**

<table>
<tr><td colspan="2" align="center">**INTERNATIONAL SEARCH REPORT**</td><td>International application No.<br>PCT/JP2009/068302</td></tr>
<tr><td colspan="3">C (Continuation). DOCUMENTS CONSIDERED TO BE RELEVANT</td></tr>
<tr><td>Category*</td><td>Citation of document, with indication, where appropriate, of the relevant passages</td><td>Relevant to claim No.</td></tr>
<tr><td align="center">A</td><td>JP 2008-088149 A (Nippon Shokubai Co., Ltd.),<br>17 April 2008 (17.04.2008),<br>entire text<br>& WO 2007/058221 A1 & JP 2007-137785 A</td><td align="center">1-8</td></tr>
<tr><td align="center">A</td><td>JP 10-087308 A (Nippon Shokubai Co., Ltd.),<br>07 April 1998 (07.04.1998),<br>entire text<br>& US 6261988 B1</td><td align="center">1-8</td></tr>
</table>

Form PCT/ISA/210 (continuation of second sheet) (April 2007)

| **INTERNATIONAL SEARCH REPORT** | International application No. |
|---|---|
| | PCT/JP2009/068302 |

**Box No. II      Observations where certain claims were found unsearchable (Continuation of item 2 of first sheet)**

This international search report has not been established in respect of certain claims under Article 17(2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III      Observations where unity of invention is lacking (Continuation of item 3 of first sheet)**

This International Searching Authority found multiple inventions in this international application, as follows:
    Documents 1 and 2 shown below, which are cited in the international search report, disclose a glycerin dehydration catalyst as defined in claim 1. Therefore, the invention of claim 1 is not novel over the inventions disclosed in Documents 1 and 2 and has no special technical feature.
    Then, examination is made on the special technical features of the claims depending on claim 1, and it is found that the inventions of claims 1-4 are classified as the main group of inventions and the inventions of other claims are classified as another group of inventions.
    (continued to extra sheet)

1. ☐ As all required additional search fees were timely paid by the applicant, this international search report covers all searchable claims.

2. ☒ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**      ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2007)

| INTERNATIONAL SEARCH REPORT | International application No. |
|---|---|
| | PCT/JP2009/068302 |

Continuation of Box No.III of continuation of first sheet(2)

Document 1: WO 2008/066079 A1 (Nippon Shokubai Co., Ltd.) 05 June 2008 (05.06.2008)
Document 2: JP 2005-213225 A (Nippon Shokubai Co., Ltd.) 11 August 2005 (11.08.2005)

Form PCT/ISA/210 (extra sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2558520 A
- JP 2006290815 A
- JP 6211724 A
- WO 2007058221 A
- WO 20061087 A
- WO 2006087084 A
- JP 54030114 A
- JP 57024643 A
- JP 58114735 A
- JP 59012759 A
- JP 159145046 A
- JP 4271841 A
- JP 7171398 A
- JP 9052049 A
- JP 10087308 A
- JP 57008761 B
- JP 57032110 A
- JP 2005213225 A

**Non-patent literature cited in the description**

- **E. Bordes ; P. Courtine.** *J. Catal.,* 1979, 57236-252
- **Burnett et al.** *Catal. Today,* 1987, 1537
- *Catalysis Today,* 1997, vol. 33, 161-171
- *Chem. Mater.,* 2002, vol. 14, 3882-3888
- **Toshio OKUHARA.** *Applied catalysis A: General,* 2006, vol. 297, 73-80